(19) Europäisches Patentamt
European Patent Office
Office européen des brevets

(11) Veröffentlichungsnummer: **0 609 733 A2**

# (12) EUROPÄISCHE PATENTANMELDUNG

(21) Anmeldenummer: **94100937.5**

(22) Anmeldetag: **24.01.94**

(51) Int. Cl.5: **C07D 239/47**, A01N 47/36, C07D 239/52, C07D 239/56, C07D 251/16, C07D 251/46, C07D 239/42, C07D 251/22

(30) Priorität: **04.02.93 DE 4303130**

(43) Veröffentlichungstag der Anmeldung: **10.08.94 Patentblatt 94/32**

(84) Benannte Vertragsstaaten: **BE CH DE DK ES FR GB IT LI NL**

(71) Anmelder: **BAYER AG**
**D-51368 Leverkusen(DE)**

(72) Erfinder: **Riebel, Hans-Jochem, Dr.**
**In der Beek 92**
**D-42113 Wuppertal(DE)**
Erfinder: **Santel, Hans-Joachim, Dr.**
**Grünstrasse 9a**
**D-51371 Leverkusen(DE)**
Erfinder: **Dollinger, Markus, Dr.**
**Hüschelrath 7**
**D-74299 Leichlingen(DE)**

## (54) Selektive Herbizide auf Basis von N-Azinyl-N'-(2-ethylthiophenylsulfonyl)-harnstoffen.

(57) Die Erfindung betrifft selektive Herbizide auf Basis von N-Azinyl-N'-(2-ethylthio-phenylsulfonyl)-harnstoffen der allgemeinen Formel (I)

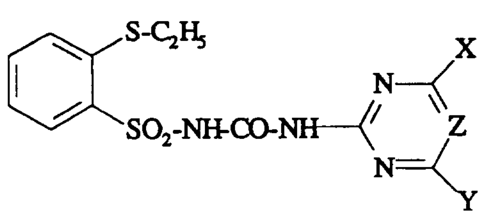

(I)

in welcher

X für Wasserstoff, Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht,

Y für Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht, und

Z für Stickstoff oder die Gruppierung C-$Z^1$ steht, worin

$Z^1$ für Wasserstoff, Fluor, Chlor, Brom, Methyl, Ethyl, Methoxy oder Ethoxy steht,

sowie neue Verbindungen der Formel (I) und Verfahren zu deren Herstellung.

EP 0 609 733 A2

Die vorliegende Erfindung betrifft die Verwendung von teilweise bekannten N-Azinyl-N'-(2-ethylthio-phenylsulfonyl)-harnstoffen als selektive Herbizide sowie bestimmte neue N-Azinyl-N'-(2-ethylthio-phenylsufonyl)-harnstoffe.

Verschiedene N-Pyrimidinyl- bzw. N-Triazinyl-N'-(2-alkylthio-phenylsulfonyl)-harnstoffe sind als potentielle Herbizide bekannt geworden, haben jedoch als Mittel zur selektiven Unkrautbekämpfung keine praktische Bedeutung erlangt (vgl. US-P 4127405, US-P 4169719, EP-A 35893, JP-A 61060667 - zitiert in Chem. Abstracts 105:172513x).

Es wurde nun gefunden, daß N-Azinyl-N'-(2-ethylthio-phenylsulfonyl)-harnstoffe der allgemeinen Formel (I)

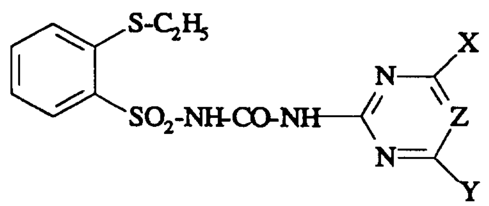

(I)

in welcher

X für Wasserstoff, Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht,

Y für Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht, und

Z für Stickstoff oder die Gruppierung C-$Z^1$ steht, worin

$Z^1$ für Wasserstoff, Fluor, Chlor, Brom, Methyl, Ethyl, Methoxy oder Ethoxy steht,

sehr gut zur selektiven Bekämpfung von Unkräutern in Kulturpflanzen geeignet sind.

Die erfindungsgemäß zu verwendenden N-Azinyl-N'-(2-ethylthio-phenylsulfonyl)-harnstoffe sind durch die Formel (I) allgemein definiert. Vorzugsweise stehen in Formel (I)

X für Wasserstoff, Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino,

Y für Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino, und

Z für Stickstoff oder die Gruppierung C-$Z^1$, worin

$Z^1$ für Wasserstoff, Fluor oder Chlor steht.

Eine besonders bevorzugte Gruppe von erfindungsgemäß zu verwendenden Verbindungen der Formel (I) sind diejenigen, bei denen

X für Chlor, Methyl oder Methoxy steht,

Y für Methyl oder Methoxy steht und

Z für eine CH-Gruppierung steht.

Eine weitere besonders bevorzugte Gruppe von erfindungsgemäß zu verwendenden Verbindungen der Formel (I) sind diejenigen, bei denen

X für Methyl oder Methoxy steht,

Y für Methyl oder Methoxy steht und

Z für Stickstoff steht.

Beispiele für die erfindungsgemäß Zu verwendenden Verbindungen der Formel (I) sind in der nachstehenden Tabelle 1 aufgeführt.

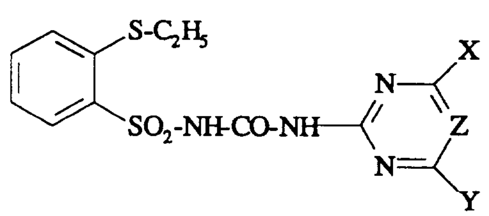

(I)

Tabelle 1

| Beispiele für die Verbindungen der Formel (I) | | | | |
|---|---|---|---|---|
| Bsp.-Nr. | X | Y | Z | Schmelzpunkt (°C) |
| 1 | $OCH_3$ | $OCH_3$ | N | 167 |
| 2 | $CH_3$ | $OCH_3$ | N | 169 |
| 3 | $OCH_3$ | $OCH_3$ | CH | 193 |
| 4 | $CH_3$ | $CH_3$ | CH | 187 |
| 5 | $CH_3$ | $OCH_3$ | CH | 190 |
| 6 | Cl | $OCH_3$ | CH | 162 |

Die N-Azinyl-N'-(2-ethylthio-phenylsulfonyl)-harnstoffe der Formel (I) sind mit Ausnahme von N-(4,6-Dimethoxy-s-triazin-2-yl)-N'-(2-ethylthio-phenylsulfonyl)-harnstoff (vgl. JP-A 61060667 - zitiert in Chem. Abstracts 105:172513x) noch nicht aus der Literatur bekannt und sind als neue Stoffe Gegenstand der vorliegenden Anmeldung.

Als neue Stoffe werden insbesondere in Tabelle 1 als Beispiele 2, 3, 4, 5 und 6 aufgeführten Verbindungen beansprucht.

Als besonders bevorzugte neue Stoffe seien die folgenden Verbindungen der Formel (I) genannt: N-(4,6-Dimethoxy-pyrimidin-2-yl)-N'-(2-ethylthio-phenylsulfonyl)-harnstoff, N-(4,6-Dimethyl-pyrimidin-2-yl)-N'-(2-ethylthio-phenylsulfonyl)-harnstoff, N-(4-Methoxy-6-methyl-pyrimidin-2-yl)-N'-(2-ethylthio-phenylsulfonyl)-harnstoff und N-(4-Chlor-6-methoxy-pyrimidin-2-yl)-N'-(2-ethylthio-phenylsulfonyl)-harnstoff.

Man erhält die N-Azinyl-N'-(2-ethylthio-phenylsulfonyl)-harnstoffe der Formel (I), wenn man

(a) 2-Ethylthio-benzolsulfonamid der Formel (II)

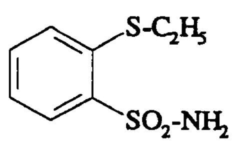

(II)

mit einem Chlorameisensäureester der allgemeinen Formel (III)

Cl-CO-O-R (III)

in welcher

R für Alkyl (insbesondere Methyl oder Ethyl), Aralkyl (insbesondere Benzyl) oder Aryl (insbesondere Phenyl) steht,

gegebenenfalls in Gegenwart eines Säureakzeptors, wie z.B. Triethylamin oder Pyridin, und gegebenenfalls in Gegenwart eines Verdünnungsmittels, wie z.B. Acetonitril, bei Temperaturen zwischen 0°C und 100°C umsetzt und

die hierbei gebildeten Sulfonylurethane (Sulfonylcarbamate) der allgemeinen Formel (IV)

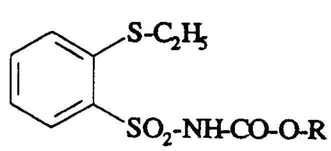

(IV)

in welcher

R die oben angegebene Bedeutung hat,

- in situ oder nach Zwischenisolierung -

mit einem Aminoazin der allgemeinen Formel (V)

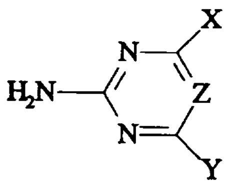 (V)

in welcher

X, Y und Z die oben angegebene Bedeutung haben,

und mit einer Säure, wie z.B. Methansulfonsäure, gegebenenfalls in Gegenwart eines Verdünnungsmittels, wie z.B. Acetonitril, bei Temperaturen zwischen 0 °C und 100 °C umsetzt (vgl. die Herstellungsbeispiele), oder wenn man

(b) 2-Ethylthio-phenylsulfonylisocyanat der Formel (VI)

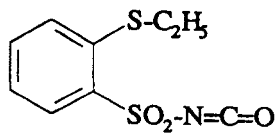 (VI)

mit einem Aminoazin der allgemeinen Formel (V)

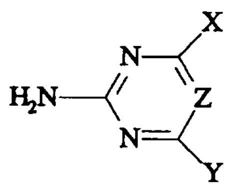 (V)

in welcher

X, Y und Z die oben angegebene Bedeutung haben,

gegebenenfalls in Gegenwart eines Verdünnungsmittels, wie z.B. Acetonitril, bei Temperaturen zwischen 20 °C und 120 °C umsetzt (vgl. die Herstellungsbeispiele).

Die Ausgangsstoffe der Formeln (II), (III), (V) und (VI) sind bekannt und/oder können nach an sich bekannten Verfahren hergestellt werden (vgl. JP-A 61060667 - zitiert in Chem. Abstracts 105:172513x; Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylormamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiwerßhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 5 g und 250 g Wirkstoff pro Hektar Bodenfläche, vorzugsweise zwischen 15 g und 150 g pro ha.

Die Herstellung und die Verwendung der erfindungsgemäßen Wirkstoffe geht aus den nachfolgenden Beispielen hervor.

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)
100% = totale Vernichtung

Eine deutliche Überlegenheit in der Wirksamkeit gegenüber dem Stand der Technik bei sehr guter Nutzpflanzenselektivität, insbesondere gegenüber Weizen, zeigen in diesem Test z.B. die Verbindungen der Beispiel-Nummern 2 und 3 in Tabelle 1.

<u>Tabelle A1:</u> Pre-emergence-Test/Gewächshaus

| Wirkstoff | Aufwand-menge (g/ha) | Weizen | Lolium | Setaria | Polygonum |
|---|---|---|---|---|---|
| 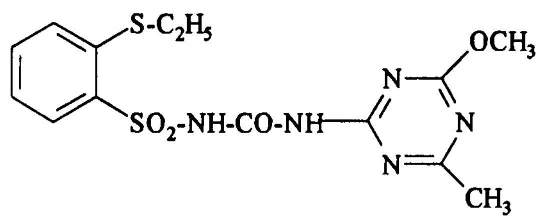 (2) | 125 | 0 | 90 | 90 | 70 |
| 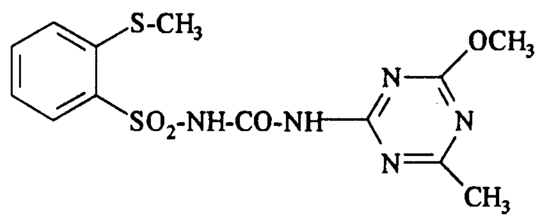 (bekannt aus US-P 4127405) | 125 | 30 | 80 | 30 | 0 |

Tabelle A2: Pre-emergence-Test/Gewächshaus

| Wirkstoff | Aufwand-menge (g/ha) | Weizen | Digitaria | Datura | Helian-thus | Sinapis |
|---|---|---|---|---|---|---|
| 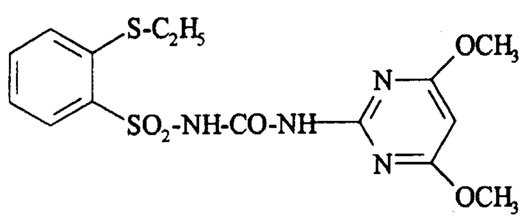 (3) | 60 | 10 | 80 | 90 | 90 | 95 |
| 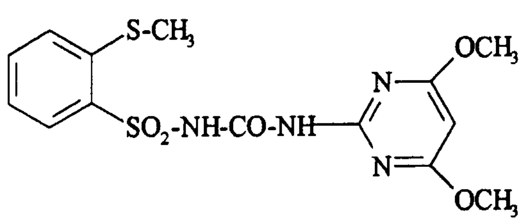 (bekannt aus JP-A 61060667) | 60 | 50 | 60 | 70 | 80 | 80 |

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)
100 % = totale Vernichtung

Eine deutliche Überlegenheit in der Wirksamkeit gegenüber dem Stand der Technik bei sehr guter Nutzpflanzenselektivität, insbesondere gegenüber Weizen, zeigen in diesem Test z.B. die Verbindungen der Beispiel-Nummern 1, 2, 3, 4 und 5 in Tabelle 1.

<u>Tabelle B1:</u> Post-emergence-Test/Gewächshaus

| Wirkstoff | Aufwandmenge (g/ha) | Weizen | Datura | Galinsoga | Sida | Stellaria | Xanthium |
|---|---|---|---|---|---|---|---|
| 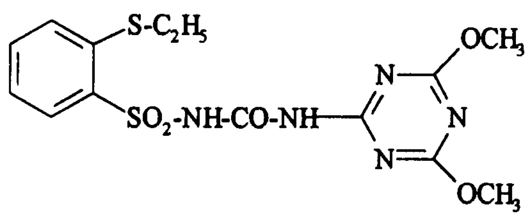 (1) | 125 | 0 | 100 | 100 | 90 | 90 | 95 |
| 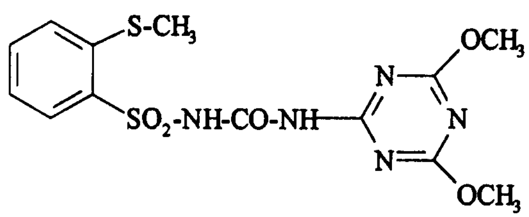 (bekannt aus JP-A 61060667) | 125 | 0 | 80 | 30 | 70 | 30 | 40 |

Tabelle B2: Post-emergence-Test/Gewächshaus

| Wirkstoff | Aufwand-menge (g/ha) | Weizen | Alopecurus | Echinochloa | Setaria |
|---|---|---|---|---|---|
| 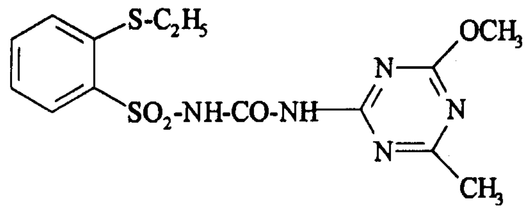 (2) | 125 | 0 | 80 | 95 | 90 |
| 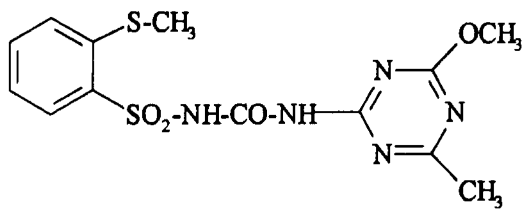 (bekannt aus US-P 4127405) | 125 | 80 | 50 | 80 | 80 |

Tabelle B3: Post-emergence-Test/Gewächshaus

| Wirkstoff | Aufwand-menge (g/ha) | Weizen | Datura | Galium | Solanum |
|---|---|---|---|---|---|
| 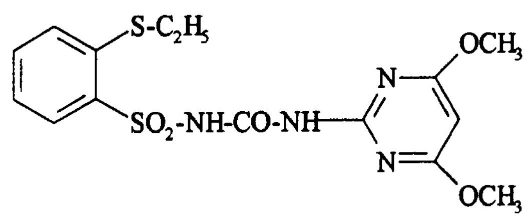 (3) | 15 | 0 | 100 | 95 | 80 |
| 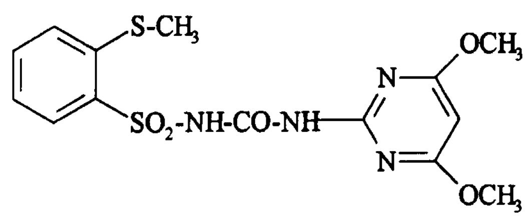 (bekannt aus JP-A 61060667) | 15 | 40 | 70 | 80 | 40 |

Tabelle B4: Post-emergence-Test/Gewächshaus

| Wirkstoff | Aufwand-menge (g/ha) | Weizen | Abuti-lon | Datura | Galin-soga | Matri-caria | Xan-thium |
|---|---|---|---|---|---|---|---|
| 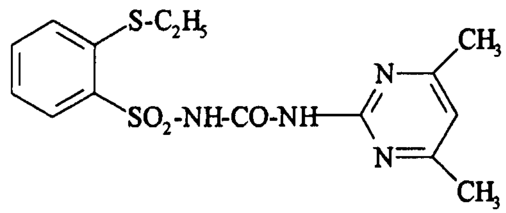 (4) | 30 | 0 | 100 | 90 | 95 | 90 | 95 |
| 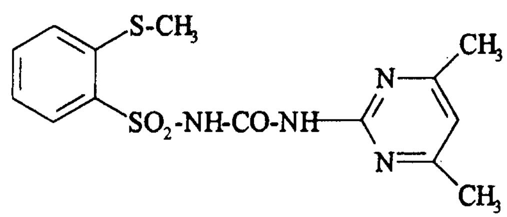 (bekannt aus JP-A 61060667) | 125 | 50 | 80 | 40 | 80 | 80 | 50 |

Tabelle B5: Post-emergence-Test/Gewächshaus

| Wirkstoff | Aufwandmenge (g/ha) | Weizen | Datura | Sida | Xanthium |
|---|---|---|---|---|---|
| 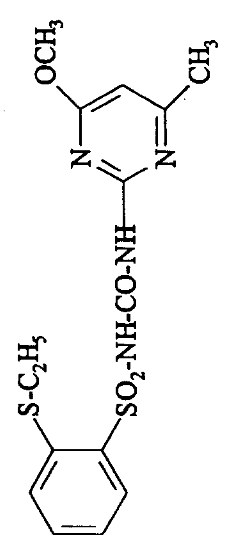 (5) | 30 | 0 | 95 | 95 | 100 |
| 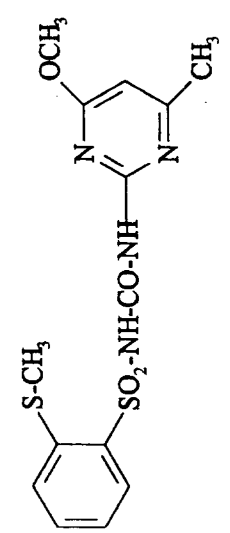 (bekannt aus US-P 4169719) | 125 | 50 | 70 | 70 | 60 |

Herstellungsbeispiele:

Herstellung der in Tabelle 1 als Beispiel 5 aufgeführten Verbindung:

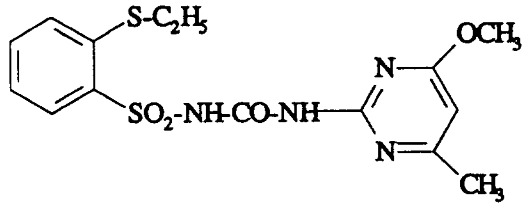

16 g (0,1 Mol) Chlorameisensäure-phenylester werden bei 20 °C bis 35 °C unter Rühren zu einer Mischung aus 21,8 g (0,1 Mol) 2-Ethylthio-benzolsulfonamid, 22 g Triethylamin und 200 ml Acetonitril gegeben und die Mischung wird 30 Minuten gerührt. Dann werden nacheinander 13,9 g (0,1 Mol) 2-Amino-4-methoxy-6-methyl-pyrimidin und 10 g Methansulfonsäure dazu gegeben. Das Reaktionsgemisch wird 60 Minuten bei 20 °C bis 30 °C gerührt, dann mit Wasser langsam auf etwa das doppelte Volumen verdünnt und das kristalline Produkt durch Absaugen isoliert .

Man erhält 24,1 g (63% der Theorie) N-(4-Methoxy-6-methyl-pyrimidin-2-yl)-N'-(2-ethylthio-phenylsulfonyl)-harnstoff vom Schmelzpunkt 190 °C.

Herstellung der in Tabelle 1 als Beispiel 2 aufgeführten Verbindung:

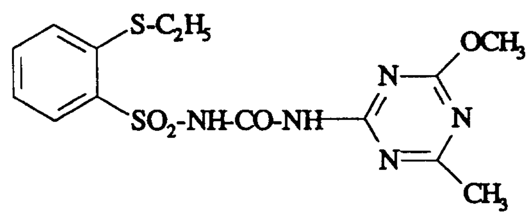

Eine Mischung aus 14 g (0,1 Mol) 2-Amino-4-methoxy-6-methyl-s-triazin, 40 g (0,16 Mol) 2-Ethylthio-phenylsulfonylisocyanat und 200 ml Acetonitril wird 3 Stunden bei 80 °C gerührt. Nach Abkühlen auf Raumtemperatur (ca. 20 °C) wird das kristallin angefallene Produkt durch Absaugen isoliert

Man erhält 28,8 g (75% der Theorie) N-(4-Methoxy-6-methyl-s-triazin-2-yl)-N'-(2-ethylhio-phenylsulfonyl)-harnstoff vom Schmelzpunkt 169 °C.

Herstellung von 2-Ethylthio-phenylsulfonylisocyanat:

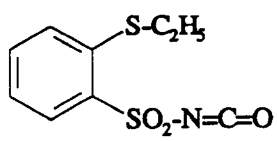

50 ml Chlorsulfonylisocyanat werden bei 90 °C unter Rühren zu einer Mischung aus 87 g (0,4 Mol) 2-Ethylthio-benzolsulfonamid und 300 mi Chlorbenzol gegeben und das komplette Gemisch wird dann 3 Stunden bei 110 °C gerührt. Nach Abkühlen wird filtriert und das Filtrat unter vermindertem Druck sorgfältig eingeengt.

Man erhält 77,8 g (80% der Theorie) 2-Ethylthio-phenylsulfonylisocyanat (Rohprodukt, welches direkt zur Umsetzung gemäß Beispiel 2 eingesetzt, aber auch durch Destillation gereinigt werden kann), Siedepunkt: 147 °C (bei 0,8 mbar).

## Patentansprüche

1. Selektiv-herbizide Mittel, gekennzeichnet durch einen Gehalt an mindestens einem N-Azinyl-N'-(2-ethylthio-phenylsulfonyl)-harnstoff der Formel (I)

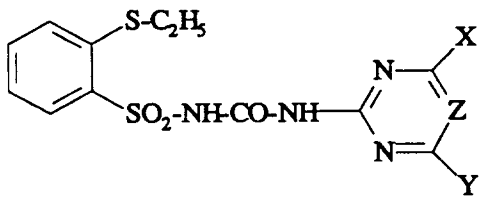

(I)

in welcher

X für Wassserstoff, Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht,

Y für Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht, und

Z für Stickstoff oder die Gruppierung C-$Z^1$ steht, worin

$Z^1$ für Wasserstoff, Fluor, Chlor, Brom, Methyl, Ethyl, Methoxy oder Ethoxy steht.

**2.** Selektiv-herbizide Mittel gemäß Anspruch 1, gekennzeichnet durch einen Gehalt an einem N-Azinyl-N'-(2-ethylthio-phenylsulfonyl)-harnstoff der Formel (I), in welcher

X für Wasserstoff, Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino steht,

Y für Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino steht und

Z für Stickstoff oder die Gruppierung C-$Z^1$ steht, worin

$Z^1$ für Wasserstoff, Fluor oder Chlor steht

**3.** Selektiv-herbizide Mittel gemäß Anspruch 1, gekennzeichnet durch einen Gehalt an einem N-Azinyl-N'-(2-ethylthio-phenylsulfonyl)-harnstoff der Formel (I), In welcher

X für Chlor, Methyl oder Methoxy steht,

Y für Methyl oder Methoxy steht und

Z für eine CH-Gruppierung steht.

**4.** Selektiv-herbizide Mittel gemäß Anspruch 1, gekennzeichnet durch einen Gehalt an einem N-Azinyl-N'-(2-ethylthio-phenylsulfonyl)-harnstoff der Formel (I), in welcher

X für Methyl oder Methoxy steht,

Y für Methyl oder Methoxy steht und

Z für Stickstoff steht.

**5.** Verfahren zur selektiven Unkrautbekämpfung in Kulturpflanzen, dadurch gekennzeichnet, daß man N-Azinyl-N'-(2-ethylthio-phenylsulfonyl)-harnstoffe der Formel (I) gemäß Anspruch 1 auf die Unkräuter und/oder ihren Lebensraum einwirken läßt.

**6.** Verwendung von N-Azinyl-N'-(2-ethylthio-phenylsulfonyl)-harnstoffen der Formel (I) gemäß Anspruch 1 zur selektiven Bekämpfung von Unkräutern in Kulturpflanzen.

**7.** Verfahren zur Herstellung von selektiv-herbiziden Mitteln, dadurch gekennzeichnet, daß man N-Azinyl-N'-(2-ethylthio-phenylsulfonyl)-harnstoffe der Formel (I) gemäß Anspruch 1 mit Streckmitteln und/oder oberflächenaktiven Mitteln vermischt.

**8.** Neue N-Azinyl-N'-(2-ethylthio-phenylsulfonyl)-harnstoffe der allgemeinen Formel (I)

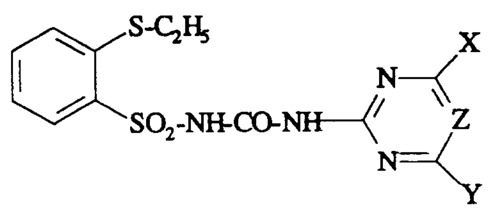

(I)

in welcher

X für Wasserstoff, Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht,

Y für Halogen, $C_1$-$C_3$-Alkyl (welches gegebenenfalls durch Fluor, Chlor, Methoxy oder Ethoxy substituiert ist), $C_1$-$C_3$-Alkoxy (welches gegebenenfalls durch Fluor, Chlor, Methoxy oder Ethoxy substituiert ist), $C_1$-$C_3$-Alkylthio, $C_1$-$C_3$-Alkylamino oder Di-($C_1$-$C_2$-alkyl)-amino steht, und

Z für Stickstoff oder die Gruppierung C-$Z^1$ steht, worin

$Z^1$ für Wasserstoff, Fluor, Chlor, Brom, Methyl, Ethyl, Methoxy oder Ethoxy steht,

mit Ausnahme der Verbindung N-(4,6-Dimethoxy-s-triazin-2-yl)-N'-(2-ethylthio-phenylsulfonyl)-harnstoff.

**9.** Verfahren zur Herstellung von N-Azinyl-N'-(2-ethylthio-phenylsulfonyl)-harnstoffen der Formel (I) gemäß Anspruch 8, dadurch gekennzeichnet, daß man

(a) 2-Ethylthio-benzolsulfonamid der Formel (II)

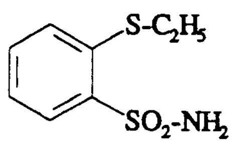

(II)

mit einem Chlorameisensäureester der allgemeinen Formel (III)

Cl-CO-O-R (III)

in welcher

R für Alkyl (insbesondere Methyl oder Ethyl), Aralkyl (insbesondere Benzyl) oder Aryl (insbesondere Phenyl) steht,

gegebenenfalls in Gegenwart eines Säureakzeptors und gegebenenfalls in Gegenwart eines Verdünnungsmittels bei Temperaturen zwischen 0 °C und 100 °C umsetzt und

das hierbei gebildete Sulfonylurethan (Sulfonylcarbamat) der allgemeinen Formel (IV)

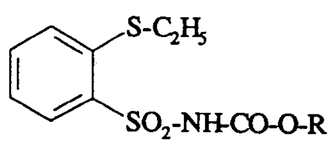

(IV)

in welcher

R die oben angegebene Bedeutung hat,

- in situ oder nach Zwischenisolierung -

mit einem Aminoazin der allgemeinen Formel (V)

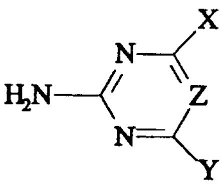

(V)

in welcher

X, Y und Z die oben angegebene Bedeutung haben,

mit einer Säure gegebenenfalls in Gegenwart eines Verdünnungsmittels bei Temperaturen zwischen 0 °C und 100 °C umsetzt oder daß man

(b) 2-Ethylthio-phenylsulfonylisocyanat der Formel (VI)

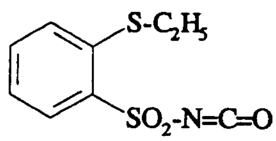

(VI)

mit einem Aminoazin der allgemeinen Formel (V)

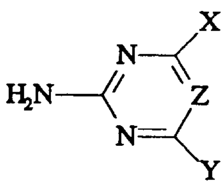

(V)

in welcher

X, Y und Z die oben angegebene Bedeutung haben,

gegebenenfalls in Gegenwart eines Verdünnungsmittels bei Temperaturen zwischen 20 °C und 120 °C umsetzt.

**10.** Die Verbindungen N-(4,6-Dimethoxy-pyrimidin-2-yl)-N'-(2-ethylthio-phenylsulfonyl)-harnstoff, N-(4,6-Dimethyl-pyrimidin-2-yl)-N'-(2-ethylthio-phenylsulfonyl)-harnstoff, N-(4-Methoxy-6-methyl-pyrimidin-2-yl)-N'-(2-ethylthio-phenylsulfonyl)-harnstoff und N-(4-Chlor-6-methoxy-pyrimidin-2-yl)-N'-(2-ethylthio-phenylsulfonyl)-harnstoff gemäß Anspruch 8.